# NEW EUROPEAN PATENT SPECIFICATION

(11) **EP 2 790 657 B2**
(45) Date of publication and mention of the opposition decision: **23.11.2022**
(45) Mention of the grant of the patent: 08.11.2017
(21) Application number: 12798310.4
(22) Date of filing: 10.12.2012
(51) Int. Cl.: A61K 8/44, A61K 8/92, A61Q 1/10, A61K 8/55, A61K 8/49, A61K 8/31, A61Q 1/00

(54) **COSMETIC COMPOSITION FOR COATING KERATINOUS FIBRES**
KOSMETISCHE ZUSAMMENSETZUNG ZUR BESCHICHTUNG VON KERATINFASERN
COMPOSITION COSMETIQUE DE REVETEMENT DES FIBRES KERATINIQUES

(30) Priority: 14.12.2011 FR 1161598; 19.12.2011 US 201161577109 P
(43) Date of publication of application: 22.10.2014
(73) Proprietor: L'OREAL, 75008 Paris (FR)
(72) Inventor: ILEKTI, Philippe, F-94700 Maisons-Alfort (FR); MOUJAHED, Zohra, F-91600 Savigny-sur-Orge (FR)
(74) Representative: L'Oreal
(86) International application number: PCT/EP2012/074994
(87) International publication number: WO 2013/087586

(56) References cited:
- EP-A1- 0 227 012
- EP-A1- 0 245 756
- EP-A2- 0 265 702
- WO-A2-2010/105951
- FR-A1- 2 908 298
- FR-A1- 2 908 300
- US-A- 5 362 418
- DATABASE GNPD [Online] MINTEL; 1 November 2011 (2011-11-01), "False Lashes Extreme Black Mascara", XP002683940, Database accession no. 1666832
- DATABASE GNPD [Online] MINTEL; 1 July 2011 (2011-07-01), "Lash Miracle Curling Mascara", XP002683941, Database accession no. 1591989

## Description

The present invention relates to a cosmetic composition for coating keratinous fibres, in particular the eyelashes or eyebrows. In particular, the said cosmetic composition is a composition for making up and optionally caring for the eyelashes. The present invention also re ates to a method for coating keratinous fibres, in particular to a method for making up and optionally caring for the eyelashes.

The composition used may be provided in particular in the form of an eyelash product, such as a mascara, or an eyebrow product. More preferentially, the invention relates to a mascara. The term "mascara" should be understood as meaning a composition intended to be applied to the eyelashes: it can be an eyelash make-up composition, an eyelash make-up base (also known as a base coat), a composition to be applied over a mascara, also known as a top coat, or a composition for the cosmetic treatment of the eyelashes. The mascara is more particularly intended for human eyelashes but also for false eyelashes.

Mascaras are prepared in particular according to two types of formulation: water-based mascaras, known ascream mascaras, in the form of a dispersion of waxes in water; anhydrous mascaras or mascaras with a low watercontent, known as waterproof mascaras, in the form of dispersions of waxes in organic solvents. The present patent application relates more specifically to "water-based" mascaras.

Compositions for coating keratinous fibres with such a type of mascara generally consist of at least one fatty phase generally formed of one or more waxes dispersed in an aqueous liquid phase by means of an emulsifying system or conveyed in an organic solvent.

The application of mascara is targeted in particular at increasing the volume of the eyelashes and consequently at increasing the intensity of the gaze. Numerous thickening or volumizing mascaras exist to do this, the principle of which consists in depositing the maximum amount of material onto the eyelashes so as to obtain this volumizing (or loading) effect. It is in particular by means of the amount of solid particles that the desired application specificities for the compositions can be adjusted, such as, for example, their fluidity or consistency, and also their thickening power (also known as the loading or making-up power). These solid particles are dispersed in the cream mascara by means of an emulsifying system composed of one or more surfactant(s).

However, a specific problem encountered with some surfactants, in particular ionic surfactants, is that they are generally unstable at low temperature (4°C), the latter crystallizing and thus destabilizing the emulsion, giving rise to aggregates of materials (in particular waxes) on the eyelashes.

An aim of the present patent application is more particularly to provide a stable mascara exhibiting a texture which is sufficiently thick to obtain a loading deposited layer, of satisfactory consistency, enabling easy application to the eyelashes and an even deposited layer, that is to say a layer which is smooth and homogeneous, even after being stored at 4°C for two months.

Another aim of the present patent application is to provide a stable mascara exhibiting a texture which is sufficiently thick to obtain a loading deposited layer, of satisfactory consistency, enabling easy application to the eyelashes and an even deposited layer, that is to say a layer which is smooth and homogeneous, even after being stored at 45°C for two months.

An aim of the present patent application is more particularly to provide a stable mascara exhibiting a texture which is sufficiently thick to obtain a loading deposited layer, of satisfactory consistency, enabling easy application to the eyelashes and an even deposited layer, that is to say a layer which is smooth and homogeneous, even after being stored for two months at temperatures oscillating between 4°C and 45°C.

An aim of the present patent application is more particularly to provide a mascara in which the waxes are homogeneously dispersed.

An aim of the present patent application is more particularly to provide a mascara in which the pigments are homogeneously dispersed.

An aim of the present patent application is more particularly to provide a mascara which is pleasant to apply.

An aim of the present invention is more particularly to provide a composition for coating keratinous fibres which makes possible good separation of the eyelashes during its application, without formation of bunches of eyelashes, and while ensuring smooth and even deposition of material (without lumps of composition).

An aim of the present invention is more particularly to obtain a composition for coating keratinous fibres, preferably a mascara, which has good application properties in terms of glide and playtime.

Another aim of the present invention is to obtain a composition for coating keratinous fibres, preferably a mascara, which gives rise to a volume effect on the eyelashes.

Another aim of the present invention is to obtain a composition for coating keratinous fibres, preferably a mascara, which has a good wear property on the eyelashes.

Another aim of the present invention is to obtain a composition for coating keratinous fibres, preferably a mascara, which gives rise to a loading or covering deposited layer.

Another aim of the present invention is to obtain a composition for coating keratinous fibres, preferably a mascara, which has good lengthening properties for the eyelashes coated with such a composition.

Another aim of the present invention is to obtain a composition for coating keratinous fibres, preferably a mascara, which has good curling properties for the eyelashes coated with such a composition.

Another aim of the present invention is to obtain a composition for coating keratinous fibres, preferably a mascara, which has good black intensity, from a colorimetry and chromaticity point of view.

Another aim of the present invention is to obtain a composition for coating keratinous fibres, preferably a mascara, which has good adhesion to the eyelashes.

Consequently, a subject-matter of the present invention is a cosmetic composition for coating keratinous fibres, preferably the eyelashes, preferably a mascara composition, of the emulsion type, preferably wax(es)-in-water emulsion type, comprising:
- an aqueous phase,
- at least one wax, **wherein the wax(es) is (are) present at a total content of greater than or equal to 20% by weight, with respect to the total weight of the composition** and
- an emulsifying system comprising:
   i) at least one anionic surfactant in the salt form **chosen from C16-C18 monoalkyl phosphate,**
   ii) at least one cationic counterion, and
   iii) at least one basic amino acid,
in which the total content of basic amino acid(s) and the total content of anionic surfactant(s) in the salt form are such that the ratio by weight of the basic amino acid(s) to the anionic surfactant(s) in the salt form is greater than or equal to 0.05, preferentially ranges from 0.075 to 10, preferentially ranges from 0.085 to 2 and more preferentially still ranges from 0.1 to 0.5.

Surprisingly and unexpectedly, the inventors of the present patent application have solved this or these problem(s) by means of such a composition. In particular, a composition in accordance with the invention gives rise to a stable creamy composition with a homogeneous and even dispersion of waxes, even after 2 months, whether at 45°C or at 4°C. It appears that such a composition also exhibits a good dispersion of the pigments. Such a composition is also pleasant to apply.

Thus, bringing together one (or more) amino acid(s) having a basic nature and one (or more) anionic surfactant(s) in the salt form, instead of normally reinforcing the affinity of the cationic counterion(s) for the anionic surfactant(s), appears, on the contrary, surprisingly, to at least partially destabilize the interaction existing between the cationic counterion(s) and the anionic surfactant(s). The inventors believe that, unexpectedly, at least a portion of the cationic counterions is replaced by amino acids having a basic nature until an exchange equilibrium is reached. Conversely, in the presence of amino acid(s) only having an acidic nature, the cationic counterions combine with the acid and the surfactant(s) precipitate(s). The presence of a least one basic amino acid appears, in any case, to interfere with the crystallization of the surfactant, which for this reason is more effective, as more available, in emulsifying the waxes.

According to a second aspect, another subject-matter of the present invention is a method for coating keratinous fibres, in particular for making up the eyelashes, comprising a stage of applying a cosmetic composition for coating keratinous fibres as described above.

According to specific preferred embodiments of the present invention:
- the emulsion is of the wax(es)-in-water type;
- the said composition comprises a fatty phase dispersed in the aqueous phase;
- the fatty phase predominantly comprises waxes;
- the said composition comprises one or more wax(es), preferably several;
- this or these wax(es) is (are) chosen from beeswax, lanolin wax, Chinese insect waxes, rice wax, carnauba wax, candelilla wax, ouricury wax, esparto grass wax, cork fibre wax, sugarcane wax, Japan wax, sumach wax, montan wax, microcrystalline waxes, paraffin wax, ozokerite, polyethylene wax, the waxes obtained by Fischer-Tropsch synthesis, a C20-C40 alkyl (hydroxystearyloxy)stearate, waxy copolymers, in particular the ethylene/vinyl acetate copolymer, and their esters, the waxes obtained by catalytic hydrogenation of animal or plant oils having linear or branched C8-C32 fatty chains, the waxes obtained by hydrogenation of olive oil esterified with stearyl alcohol, the waxes obtained by hydrogenation of castor oil esterified with cetyl alcohol, silicone waxes, such as alkyl or alkoxydimethicones having from 16 to 45 carbon atoms, fluoro waxes and their mixture(s);
- the said composition comprises at least one wax chosen from candelilla wax, carnauba wax, beeswax, paraffin wax, polyethylene wax, rice wax and their mixture(s);
- the fatty phase comprises predominantly waxes, preferably a mixture of polar wax(es) and non-polar wax(es);
- the non-polar wax(es) is (are) chosen from hydrocarbon waxes, preferably from microcrystalline waxes, paraffin waxes, ozokerite, polyethylene waxes and their mixture(s);
- the total content of polar wax(es) is greater than or equal to 7% by weight, with respect to the total weight of the composition, preferably between 10% and 40% by weight, with respect to the total weight of the composition;
- the polar wax(es) is (are) chosen from ester waxes, alcohol waxes, silicone waxes and their mixture(s);
- the said composition comprises at least:
   * one ester wax chosen from:
      i) waxes of formula R₁COOR₂ in which R₁ and R₂ represent linear, branched or cyclic aliphatic chains, the number of atoms of which varies from 10 to 50, which can comprise a heteroatom, such as O, N or P, and the melting point of which varies from 25 to 120°C;
      ii) di(1,1,1-trimethylolpropane) tetrastearate;
      iii) diester waxes of a dicarboxylic acid of general formula R₃-(-OCO-R₄-COO-R₅), in which R₃ and R₅ are identical or different, preferably identical, and represent a C4-C30 alkyl group (alkyl group comprising from 4 to 30 carbon atoms) and R₄ represents a linear or branched C4-C30 aliphatic group (alkylene group comprising from 4 to 30 carbon atoms) which may or may not comprise one or more unsaturations and which is preferably linear and unsaturated;
      iv) waxes obtained by catalytic hydrogenation of animal or vegetable oils having linear or branched C8-C32 fatty chains, and also waxes obtained by hydrogenation of castor oil esterified with cetyl alcohol, and waxes obtained by hydrogenation of olive oil esterified with stearyl alcohol; and
      v) beeswax, synthetic beeswax, polyglycerolated beeswax, carnauba wax, candelilla wax, oxypropylenated lanolin wax, rice bran wax, ouricury wax, esparto grass wax, cork fibre wax, sugarcane wax, Japan wax, sumach wax, montan wax, orange wax, laurel wax and hydrogenated jojoba wax;
      vi) and their mixture(s);
   * at least one alcohol wax, and their mixture(s);
   * at least one silicone wax, and their mixture(s);
   and the mixture of several of these waxes;
- the said composition comprises at least one polar wax, preferably at least two polar waxes, chosen from beeswax, synthetic beeswax, polyglycerolated beeswax, carnauba wax, candelilla wax, oxypropylenated lanolin wax, rice bran wax, ouricury wax, esparto grass wax, cork fibre wax, sugarcane wax, Japan wax, sumach wax, montan wax,orange wax, laurel wax, hydrogenated jojoba wax and their mixture(s);
- the total content of non-polar wax(es) and the total content of polar wax(es) are such that the ratio by weight of nonpolar wax(es) to the polar wax(es) is greater than or equal to 1/3;
- the said composition comprises at least 20% by weight of non-polar wax(es), preferably at least 40% by weight of non-polar wax(es), with respect to the total weight of wax(es);
- the total content of wax(es) is greater than or equal to 20% by weight, with respect to the total weight of the composition, preferably between 22% and 40% by weight, with respect to the total weight of the composition;
- the said emulsifying system is chosen from C16-C18, monoalkyl phosphate;
- the said emulsifying system comprises at least one monocetyl phosphate;
- the said anionic surfactant(s) is (are) present in a total content of greater than or equal to 1% by weight, with respect to the total weight of the composition, preferably ranging from 1.5% to 15% by weight and better still from 2% to 10% by weight, with respect to the total weight of the composition;
- the said cationic counterion(s) is (are) chosen from a cation of inorganic origin, in particular chosen from alkali metal cations, or a cation of organic origin, preferably of inorganic origin;
- the said cationic counterion(s) is (are) chosen from a cation of inorganic origin of alkali metals;
- the said alkali metal is (are) chosen from sodium and potassium, preferably potassium;
- the total content of cationic counterion(s) is greater than or equal to 0.05% by weight, preferably inclusively between 0.08% and 3% by weight and better still between 0.5% and 2% by weight, with respect to the total weight of the composition;
- the total content of anionic surfactant(s) and the total content of wax(es) are such that the ratio by weight of anionic surfactant(s) to the wax(es) is greater than or equal to 1/10, preferably between 1/8 and 1/2 inclusive;
- the basic amino acid(s) is (are) preferably chosen from arginine, histidine, lysine and their mixture(s), and preferably comprises arginine;
- the total content of basic amino acid(s) is greater than or equal to 0.1% by weight, with respect to the total weight of the composition, and preferentially ranges from 0.5% to 4% by weight, with respect to the total weight of the composition;
- the cosmetic composition does not comprise C16-C22 fatty acid in the acid form, in particular does not comprise stearic acid in the acid form (i.e. in a form not neutralized by a salt);
- the cosmetic composition comprises at least one hydrophilic or lipophilic film-forming polymer and/or at least one hydrophilic gelling agent;
- the said composition is devoid of oil or organic solvent;
- the cosmetic composition comprises at least one volatile or non-volatile oil, for example a hydrocarbon oil;
- the said composition comprises at least one volatile or non-volatile oil, for example a hydrocarbon oil;
- the cosmetic composition comprises at least one pulverulent colorant chosen from pigments and preferably chosen from metal oxides, in particular iron oxides and titanium oxides;
- the metal oxide(s) is (are) preferably present at a content of greater than or equal to 2% by weight, with respect to the total weight of the composition, and advantageously inclusively between 3% and 15% by weight, with respect to the total weight of the composition;
- the composition exhibits a neutral or basic pH, preferably of between 7 and 9;
- the composition is a make-up composition, a make-up base, a "top coat" composition to be applied over a makeup, or a composition for cosmetically treating or caring for keratinous fibres;
- the said composition exhibits a viscosity at 20°C of between 30 and 300 poises, better still between 60 and 150 poises. The viscosity measurement is carried out at 20°C, using a Rheomat RM180 viscometer equipped with a No. 4 spindle, the measurement being carried out after rotating the spindle for 10 minutes (at the end of which time stabilization of the viscosity and of the rotational speed of the spindle are observed), at a shear rate of 200 s⁻¹.

Other characteristics, properties and advantages of the present invention will become more clearly apparent on reading the description and examples which follow.

### Aqueous phase

The composition according to the invention comprises an aqueous phase, which can form a continuous phase of the composition.

The aqueous phase comprises water. It can also comprise at least one water-soluble solvent.

In the present invention, the term "water-soluble solvent" denotes a compound which is liquid at ambient temperature (20°C) and atmospheric pressure (760 mmHg) and which is miscible with water.

The water-soluble solvents which can be used in the compositions according to the invention can in addition be volatile.

Mention may in particular be made, among the water-soluble solvents which can be used in the compositions in accordance with the invention, of lower monoalcohols having from 1 to 5 carbon atoms, such as ethanol and isopropanol, and glycols having from 2 to 8 carbon atoms, such as ethylene glycol, propylene glycol, 1,3-butylene glycol and dipropylene glycol.

The aqueous phase (water and optionally the water-miscible solvent) is generally present in the composition according to the present patent application in a content ranging from 20% to 90% by weight, with respect to the total weight of the composition, preferably ranging from 25% to 80% by weight, preferentially ranging from 30% to 70% by weight and better still ranging from 45% to 60% by weight, with respect to the total weight of the composition.

### Fatty phase

A fatty phase in accordance with the invention advantageously comprises at least one wax chosen from at least one non-polar wax, at least one polar wax and their mixture(s).

This fatty phase can also comprise constituents chosen in particular from at least one volatile oil, at least one additional non-volatile oil, pasty fatty substances and their mixture(s).

Preferably, the fatty phase is composed predominantly of waxes and more preferentially still exclusively of waxes. According to a preferred embodiment, the fatty phase comprises at least 75% by weight of wax(es) and more preferentially still at least 90% by weight of waxes, with respect to the total weight of the fatty phase.

### Wax(es)

The wax(es) under consideration in the context of the present invention is (are) generally a lipophilic compound which is solid at ambient temperature (20°C) and atmospheric pressure (760 mmHg), which exhibits a reversible solid/liquid change in state and which has a melting point of greater than or equal to 30°C which can range up to 200°C and in particular up to 120°C.

In particular, the waxes suitable for the invention can exhibit a melting point of greater than or equal to 45°C and in particular of greater than or equal to 55°C.

Within the meaning of the invention, the melting point corresponds to the temperature of the most endothermic peak observed in thermal analysis (DSC) as described in Standard ISO 11357-3; 1999. The melting point of the wax can be measured using a differential scanning calorimeter (DSC), for example the calorimeter sold under the name DSC Q2000 by TA Instruments.

Preferably, the waxes comprise at least one crystallizable part, which is visible by X-ray diffraction observations.

Preferably, the waxes exhibit an enthalpy of fusion ΔHf of greater than or equal to 70 J/g.

The measurement protocol is as follows:
A sample of 5 mg of wax placed in a crucible is subjected to a first temperature rise passing from -20°C to 120°C at a heating rate of 10°C/minute, is then cooled from 120°C to -20°C at a cooling rate of 10°C/minute and is finally subjected to a second temperature rise passing from -20°C to 120°C at a heating rate of 5°C/minute. During the second temperature rise, the following parameters are measured:
- the melting point (M.p.) of the wax, as mentioned above corresponding to the temperature of the most endothermic peak of the melting curve observed, representing the variation of the difference in power absorbed as a function of the temperature,
- ΔH_{f}: the enthalpy of fusion of the wax, corresponding to the integral of the entire melting curve obtained. This enthalpy of fusion of the wax is the amount of energy necessary to make the compound change from the solid state to the liquid state. It is expressed in J/g.

The wax(es) can be hydrocarbon waxes, fluoro waxes and/or silicone waxes and can be of vegetable, mineral, animal and/or synthetic origin.

The wax(es) can be present at a total content of greater than or equal to 20% by weight, with respect to the total weight of the composition. Preferably, it is (they are) present in a content ranging from 22% to 40% by weight, with respect to the total weight of the composition, better still from 25% to 35% by weight, with respect to the total weight of the composition.

A composition according to the invention advantageously comprises at least one non-polar wax and at least one polar wax.

### Non-polar waxes:

A composition according to the invention advantageously comprises at least one non-polar wax.

Within the meaning of the present invention, the term "non-polar wax" is understood to mean a wax for which the solubility parameter calculated beyond its melting point as defined below, δa, is equal to 0 (J/cm³).

The definition and calculation of the solubility parameters in the Hansen three-dimensional solubility space are described in the paper by C.M. Hansen: "The three-dimensional solubility parameters", J. Paint Technol., 39, 105 (1967).

According to this Hansen space:
- δD characterizes the London dispersion forces resulting from the formation of dipoles induced during molecular
   impacts;
- δp characterizes the Debye interaction forces between permanent dipoles and also the Keesom interaction forces
   between induced dipoles and permanent dipoles;
- δh characterizes the forces of specific interactions (such as hydrogen bonds, acid/base bonds, donor/acceptor bonds,
   and the like);
- δa is determined by the equation: δa= (δp² +δh²)^{1/2}

The parameters δp, δh, δD and δa are expressed as (J/cm³)^{1/2}.

Non-polar waxes are in particular hydrocarbon waxes consisting solely of carbon and hydrogen atoms and devoid of heteroatoms, such as N, O, Si and P.

In particular, the expression "non-polar wax" is understood to mean a wax which consists solely of non-polar wax and is not a mixture also comprising other types of waxes which are not non-polar waxes.

Mention may in particular be made, by way of illustration of non-polar waxes suitable for the invention, of hydrocarbon waxes, such as microcrystalline waxes, paraffin waxes, ozokerite, polyethylene waxes and their mixture(s).

Mention may be made, as polyethylene wax, of Performalene 500-L Polyethylene and Performalene 400 Polyethylene, sold by New Phase Technologies.

Mention may be made, as ozokerite, of Ozokerite Wax SP 1020 P.

Mention may be made, as microcrystalline waxes which can be used, of Multiwax W 445^{®}, sold by Sonneborn, and Microwax HW^{®} and Base Wax 30540^{®}, sold by Paramelt.

Mention may in particular be made, as microwaxes which can be used in the compositions according to the invention as non-polar wax, of polyethylene microwaxes, such as those sold under the names Micropoly 200^{®}, 220^{®}, 220L^{®} and 250S^{®} by Micro Powders.

Preferably, the composition according to the invention comprises a content of non-polar wax(es) of greater than or equal to 7% by weight, with respect to the total weight of the composition. The composition according to the invention advantageously comprises a content of non-polar wax(es) ranging from 10% to 40% by weight, with respect to the total weight of the composition, and better still from 12% to 30% by weight, with respect to the total weight of the composition.

The said composition comprises at least 20% by weight of non-polar wax(es), preferably at least 40% by weight of non-polar wax(es), with respect to the total weight of wax(es).

### Polar wax

A composition according to the invention advantageously comprises at least one polar wax.

Within the meaning of the present invention, the term "polar wax" is understood to mean a wax for which the solubility parameter calculated beyond its melting point, δa, is other than 0 (J/cm³)^{1/2}.

In particular, the term "polar wax" is understood to mean a wax for which the chemical structure is formed essentially of, indeed even consists of, carbon and hydrogen atoms and comprises at least one highly electronegative heteroatom, such as an oxygen, nitrogen, silicon or phosphorus atom.

The polar wax(es) can in particular be hydrocarbon, fluoro or silicone wax(es), preferably hydrocarbon waxes.

The term "silicone wax" is understood to mean a wax comprising at least one silicon atom, in particular comprising Si-O groups.

The term "hydrocarbon wax" is understood to mean a wax formed essentially of, indeed even consisting of, carbon and hydrogen atoms and optionally oxygen and nitrogen atoms, and which does not comprise a silicon or fluorine atom. It can comprise alcohol, ester, ether, carboxylic acid, amine and/or amide groups.

Preference is given in particular, as hydrocarbon polar wax, to a wax chosen from ester waxes and alcohol waxes, more preferentially ester waxes.

The term "ester wax" is understood to mean, according to the invention, a wax comprising at least one ester functional group. The term "alcohol wax" is understood to mean, according to the invention, a wax comprising at least one alcohol functional group, that is to say comprising at least one free hydroxyl (OH) group.

Use may in particular be made, preferably, as ester wax, of those chosen from:
i) waxes of formula R₁COOR₂ in which R₁ and R₂ represent linear, branched or cyclic aliphatic chains, the number of atoms of which varies from 10 to 50, which can comprise a heteroatom, such as O, N or P, and the melting point of which varies from 25°C to 120°C. Use may in particular be made, as ester wax, of a C20-C40 alkyl (hydroxystearyloxy) stearate (the alkyl group comprising from 20 to 40 carbon atoms), alone or as a mixture, or of a C20-C40 alkyl stearate. Such waxes are sold in particular under the names Kester Wax K 82 P^{®}, Hydroxypolyester K 82 P^{®}, Kester Wax K 80 P^{®} or Kester Wax K82H^{®} by Koster Keunen. Use may also be made of a glycol and butylene glycol montanate (octacosanoate), such as the wax Licowax KPS Flakes (INCI name: glycol montanate) sold by Clariant;
ii) di(1,1,1-trimethylolpropane) tetrastearate, sold under the name Hest 2T-4S^{®} by Heterene ;
iii) diester waxes of a dicarboxylic acid of general formula R₃-(-OCO-R₄-COO-R₅), in which R₃ and R₅ are identical or different, preferably identical, and represent a C4-C30 alkyl group (alkyl group comprising from 4 to 30 carbon atoms) and R₄ represents a linear or branched C4-C30 aliphatic group (alkylene group comprising from 4 to 30 carbon atoms) which may or may not comprise one or more unsaturations and which is preferably linear and unsaturated;
iv) mention may also be made of the waxes obtained by catalytic hydrogenation of animal or vegetable oils having linear or branched C8-C32 fatty chains, for example such as hydrogenated jojoba oil, hydrogenated sunflower oil, hydrogenated castor oil or hydrogenated coconut oil, and also the waxes obtained by hydrogenation of castor oil esterified with cetyl alcohol, such as those sold under the names Phytowax Castor 16L64^{®} and 22L73^{®} by Sophim; such waxes are described in Application FR-A-2 792 190; and the waxes obtained by hydrogenation of olive oil esterified with stearyl alcohol, such as the product sold under the name Phytowax Olive 18 L 57; or else v) beeswax, synthetic beeswax, polyglycerolated beeswax, beeswax esterified with an oxyethylene group, carnauba wax, candelilla wax, oxypropylenated lanolin wax, rice bran wax, ouricury wax, esparto grass wax, cork fibre wax, sugarcane wax, Japan wax, sumach wax, montan wax, orange wax, laurel wax or hydrogenated jojoba wax;
vi)and their mixture(s).

According to another embodiment, the polar wax can be an alcohol wax. The term "alcohol wax" is understood to mean, according to the invention, a wax comprising at least one alcohol functional group, that is to say comprising at least one free hydroxyl (OH) group.

Mention may be made, as alcohol wax, for example, of the wax Performacol 550-L Alcohol from New Phase Technologies.

According to a second embodiment, the polar wax can be a silicone wax, such as siliconized beeswax, or an alkyl dimethicone, such as the C30-C45 alkyl dimethicone sold under the reference SF1642 by Momentive Performance

### Materials

Preferably, the composition according to the invention comprises a content of polar wax(es) and advantageously of polar hydrocarbon wax of greater than or equal to 7% by weight, with respect to the total weight of the composition. The composition according to the invention advantageously comprises a content of polar wax(es) ranging from 10% to 40% by weight, with respect to the total weight of the composition, and better still from 12% to 30% by weight, with respect to the total weight of the composition.

A composition according to the invention advantageously comprises at least 50% by weight of polar wax(es), with respect to the total weight of wax(es)

The total content of non-polar wax(es) and the total content of polar wax(es) are such that the ratio by weight of non-polar wax(es) to polar wax(es) is greater than or equal to 1/3.

### Oil or organic solvent

The compositions according to the invention can comprise at least one oil or organic solvent.

The compositions according to the invention can in particular comprise at least one oil chosen from at least one non-volatile oil, at least one volatile oil and their mixture.

### Non-volatile oil

The term "oil" is understood to mean a fatty substance which is liquid at ambient temperature (20°C) and atmospheric pressure (760 mmHg).

The term "non-volatile oil" is understood to mean an oil which remains on the skin or the keratinous fibre at ambient temperature (20°C) and atmospheric pressure (760 mmHg). More specifically, a non-volatile oil exhibits an evaporation rate strictly of less than 0.01 mg/cm²/min.

In order to measure this evaporation rate, 15 g of oil or of oil mixture to be tested are introduced into a crystallizing dish with a diameter of 7 cm placed on a balance in a large chamber of approximately 0.3 m³ which is regulated in temperature, at a temperature of 25°C, and regulated in hygrometry, at a relative humidity of 50%. The liquid is allowed to freely evaporate, without stirring it, while providing ventilation by means of a fan (Papst-Motoren, reference 8550 N, rotating at 2700 revolutions per minute) placed in a vertical position above the crystallizing dish containing the said oil or the said mixture, the blades being directed towards the crystallizing dish and at a distance of 20 cm with respect to the bottom of the crystallizing dish. The mass of oil remaining in the crystallizing dish is measured at regular intervals. The evaporation rates are expressed in mg of oil evaporated per unit of surface area (cm²) and per unit of time (minute).

The said at least one non-volatile oil can be chosen from hydrocarbon oils and silicone oils, and their mixtures, preferably from hydrocarbon oils.

The non-volatile hydrocarbon oils suitable for the present invention can be chosen in particular from:
- hydrocarbon oils of vegetable origin, such as triglycerides composed of esters of fatty acids and of glycerol, the fatty acids of which can have varied chain lengths from C4 to C28, it being possible for the latter to be linear or branched and saturated or unsaturated; these oils are in particular wheat germ oil, sunflower oil, grape seed oil, sesame oil, maize oil, apricot oil, castor oil, shea oil, avocado oil, olive oil, soybean oil, sweet almond oil, rapeseed oil, cottonseed oil, hazelnut oil, macadamia oil, jojoba oil, palm oil, alfalfa oil, poppy oil, pumpkinseed oil, cucumber oil, blackcurrant oil, evening primrose oil, millet oil, barley oil, quinoa oil, rye oil, safflower oil, candlenut oil, passionflower oil and musk rose oil; or alternatively caprylic/capric acid triglycerides, such as those sold by Stéarineries Dubois or those sold under the names Miglyol 810^{®}, 812^{®} and 818^{®} by Sasol;
- synthetic ethers having from 10 to 40 carbon atoms;
- linear or branched hydrocarbons of mineral or synthetic origin, other than the polymers according to the invention,
   such as petrolatum, polybutenes, polydecenes, squalane and their mixtures;
- synthetic esters, such as oils of formula R₁COOR₂ in which R₁ represents the residue of a linear or branched fatty acid comprising from 1 to 40 carbon atoms and R2 represents a hydrocarbon chain, in particular a branched hydrocarbon chain, comprising from 1 to 40 carbon atoms, provided that R₁ + R₂ ≥ 10, such as, for example, purcellin oil (cetearyl octanoate), isopropyl myristate, isopropyl palmitate, C12 to C15 alkyl benzoate, hexyl laurate, diisopropyl adipate, isononyl isononanoate, 2-ethylhexyl palmitate, isostearyl isostearate, or octanoates, decanoates or ricinoleates
   of alcohols or polyalcohols, such as propylene glycol dioctanoate; hydroxylated esters, such as isostearyl lactate or diisostearyl malate; and pentaerythritol esters;
- fatty alcohols which are liquid at ambient temperature (20°C) and atmospheric pressure (760 mmHg), with a branched and/or unsaturated carbon-based chain having from 12 to 26 carbon atoms, such as octyldodecanol, isostearyl alcohol, oleyl alcohol, 2-hexyldecanol, 2-butyloctanol or 2-undecylpentadecanol;
- higher fatty acids, such as oleic acid, linoleic acid, linolenic acid and their mixtures.

The non-volatile silicone oils suitable for the present invention can be chosen in particular from:
- non-volatile polydimethylsiloxanes (PDMSs), polydimethylsiloxanes comprising alkyl or alkoxy groups which are pendent and/or at the end of the silicone chain, which groups each have from 2 to 24 carbon atoms, or phenylated silicones, such as phenyl trimethicones, phenyl dimethicones, phenyl(trimethylsiloxy)diphenylsiloxanes, diphenyl
   dimethicones, diphenyl(methyldiphenyl)trisiloxanes or (2-phenylethyl)trimethylsiloxysilicates.

A composition according to the invention optionally comprises at least one non-volatile hydrocarbon oil of vegetable origin, such as triglycerides consisting of esters of fatty acids and of glycerol, the fatty acids of which can have varied chain lengths from C4 to C28, in particular palm oil and jojoba oil. A composition according to the invention is preferably devoid of non-volatile silicone oil(s).

A composition according to the invention is preferably devoid of non-volatile oil. However, the total content of non-volatile oil(s) in a composition in accordance with the invention can range from 0.01% to 10% by weight, in particular from 0.1% to 8% by weight and preferably from 0.25% to 5% by weight, with respect to the total weight of the composition.

According to a preferred embodiment, a composition according to the invention comprises less than 5% by weight of non-volatile oil(s), with respect to the total weight of the composition.

### Volatile oil

The composition according to the invention can comprise at least one volatile oil.

The term "volatile oil" is understood to mean an oil (or non-aqueous medium) capable of evaporating on contact with the skin in less than one hour, at ambient temperature (20°C) and atmospheric pressure (760 mmHg). The volatile oil is a volatile cosmetic oil which is liquid at ambient temperature (20°C) and atmospheric pressure (760 mmHg). More specifically, a volatile oil exhibits an evaporation rate of between 0.01 and 200 mg/cm²/min, limits included.

This volatile oil can be hydrocarbon oil.

The volatile hydrocarbon oil can be chosen from hydrocarbon oils having from 7 to 16 carbon atoms.

The composition according to the invention can comprise one or more volatile branched alkane(s). The term "one or more volatile branched alkane(s)" is understood to mean, without distinction, "one or more volatile branched alkane oil(s)".

Mention may in particular be made, as volatile hydrocarbon oil having from 7 to 16 carbon atoms, of branched C8-C16 alkanes, such as C8-C16 isoalkanes (also known as isoparaffins), isododecane, isodecane, isohexadecane, for example the oils sold under the Isopar or Permethyl trade names, branched C8-C16 esters, such as isohexyl neopentanoate, and their mixtures. Preferably, the volatile hydrocarbon oil having from 8 to 16 carbon atoms is chosen from isododecane, isodecane, isohexadecane and their mixtures, and is in particular isododecane.

The composition according to the invention can comprise one or more volatile linear alkane(s). The term "one or more volatile linear alkane(s)" is understood to mean, without distinction, "one or more volatile linear alkane oil(s)".

A volatile linear alkane suitable for the invention is liquid at ambient temperature (20°C) and atmospheric pressure (760 mmHg).

A "volatile linear alkane" suitable for the invention is understood to mean a cosmetic linear alkane which is capable of evaporating on contact with the skin in less than one hour, at ambient temperature (20°C) and atmospheric pressure (760 mmHg, that is to say 101 325 Pa), which is liquid at ambient temperature (20°C) and which has in particular an evaporation rate ranging from 0.01 to 15 mg/cm2/min, at ambient temperature (20°C) and atmospheric pressure (760 mmHg).

The linear alkanes, preferably of vegetable origin, comprise from 7 to 15 carbon atoms, in particular from 9 to 14 carbon atoms and more particularly from 11 to 13 carbon atoms. Mention may be made, as example of linear alkane suitable for the invention, of the alkanes described in Patent Applications WO 2007/068371 or WO 2008/155059 from Cognis (mixtures of distinct alkanes which differ by at least one carbon). These alkanes are obtained from fatty alcohols, themselves obtained from coconut oil or palm oil.Mention may be made, as example of linear alkane suitable for the invention, of n-heptane (C7), n-octane (C8), n-nonane (C9), n-decane (C10), n-undecane (C11), n-dodecane (C12), n-tridecane (C13), n-tetradecane (C14), n-pentadecane (C15) and their mixtures, in particular the mixture of n-undecane (C11) and n-tridecane (C13) described in Example 1 of Patent Application WO 2008/155059 of Cognis. Mention may also be made of n-dodecane (C12) and n-tetradecane (C14) sold by Sasol respectively under the references Parafol 12-97 and Parafol 14-97, and also their mixtures. Use may be made of the linear alkane alone or as a mixture of at least two distinct alkanes which differ from one another by a carbon number of at least 1, in particular a mixture of at least two distinct linear alkanes comprising from 10 to 14 carbon atoms which differ from each other by a carbon number of at least 2, especially a mixture of volatile linear C11/C13 alkanes or a mixture of linear C12/C14 alkanes, especially an n-undecane/n-tridecane mixture (such a mixture can be obtained according to Example 1 or Example 2 of WO 2008/155059).

In an alternative form or additionally, the composition prepared can comprise at least one volatile silicone oil or solvent which is compatible with a cosmetic use.

The term "silicone oil" is understood to mean an oil comprising at least one silicon atom and in particular comprising Si-O groups. According to one embodiment, the said composition comprises less than 10% by weight of volatile silicone oil(s), with respect to the total weight of the composition, better still less than 5% by weight, indeed even is devoid of volatile silicone oil.

Mention may be made, as volatile silicone oil, of cyclic polysiloxanes, linear polysiloxanes and their mixtures. Mention may be made, as volatile linear polysiloxanes, of hexamethyldisiloxane, octamethyltrisiloxane, decamethyltetrasiloxane, tetradecamethylhexasiloxane and hexadecamethylheptasiloxane. Mention may be made, as volatile cyclic polysiloxanes, of hexamethylcyclotrisiloxane, octamethylcyclotetrasiloxane, decamethylcyclopentasiloxane and dodecamethylcyclohexasiloxane.

In an alternative form or additionally, the composition prepared can comprise at least one volatile fluoro oil.

The term "fluoro oil" is understood to mean an oil comprising at least one fluorine atom.

Mention may be made, as volatile fluoro oil, of nonafluoromethoxybutane or perfluoromethylcyclopentane, and their mixtures.

A composition according to the invention is preferably devoid of volatile oil. However, at least one volatile oil can be present in a total content ranging from 0.05% to 20% by weight, with respect to the total weight of the composition, preferably ranging from 0.1% to 15% by weight and preferentially ranging from 0.1% to 10% by weight. In particular, the volatile oil can be present ranging from 0.5% to 5% by weight, with respect to the total weight of the composition.

According to a preferred embodiment, a composition according to the invention comprises less than 5% by weight of volatile oil(s), with respect to the total weight of the composition.

### Pasty fatty substances

A composition according to the invention can comprise at least one pasty fatty substance.

Within the meaning of the present invention, the term "pasty fatty substance" is understood to mean a lipophilic fatty compound exhibiting a reversible solid/liquid change in state and comprising, at a temperature of 23°C, a liquid fraction and a solid fraction.

In other words, the starting melting point of the pasty compound can be less than 23°C. The liquid fraction of the pasty compound, measured at 23°C, can represent from 9% to 97% by weight of the compound. This liquid fraction at 23°C preferably represents between 15% and 85% and more preferably between 40% and 85% by weight.

Preferably, the pasty fatty substances exhibit an end melting point of less than 60°C.

Preferably, the pasty fatty substances exhibit a hardness of less than or equal to 6 MPa.

Preferably, the pasty fatty substances exhibit, in the solid state, a crystal organization which is visible by X-ray diffraction characterizations.

Within the meaning of the invention, the melting point corresponds to the temperature of the most endothermic peak observed in thermal analysis (DSC) as described in Standard ISO 11357-3; 1999. The melting point of a pasty substance or of a wax can be measured using a differential scanning calorimeter (DSC), for example the calorimeter sold under the name DSC Q2000 by TA Instruments.

As regards the measurement of the melting point and the determination of the end melting point, the sample preparation and measurement protocols are as follows: A sample of 5 mg of pasty fatty substance, preheated to 80°C and withdrawn with magnetic stirring using a spatula which is also heated, is placed in a hermetic aluminium capsule, or crucible. Two tests are carried out to ensure the reproducibility of the results.

The measurements are carried out on the abovementioned calorimeter. The oven is flushed with nitrogen. Cooling is carried out with an RCS 90 heat exchanger. The sample is subsequently subjected to the following protocol: it is first of all placed at a temperature of 20°C, is then subjected to a first temperature rise passing from 20°C to 80°C at a heating rate of 5°C/minute, is then cooled from 80°C to -80°C at a cooling rate of 5°C/minute and, finally, is subjected to a second temperature rise passing from -80°C to 80°C at a heating rate of 5°C/minute. During the second temperature rise, the variation in the difference in power absorbed by the empty crucible and the crucible containing the sample ofpasty substance or wax is measured as a function of the temperature. The melting point of the compound is the value of the temperature corresponding to the top of the peak of the curve representing the variation in the difference in power absorbed as a function of the temperature.

The end melting point corresponds to the temperature at which 95% of the sample has melted.

The liquid fraction by weight of the pasty compound at 23°C is equal to the ratio of the enthalpy of fusion consumed at 23°C to the enthalpy of fusion of the pasty compound.

The enthalpy of fusion of the pasty compound is the enthalpy consumed by the compound in order to change from the solid state to the liquid state. The pasty compound is said to be in the solid state when all of its mass is in crystalline solid form. The pasty compound is said to be in the liquid state when all of its mass is in liquid form.

The enthalpy of fusion of the pasty compound is equal to the integral of the entire melting curve obtained using the abovementioned colorimeter, with a temperature rise of 5 or 10°C per minute, according to Standard ISO11357-3:1999. The enthalpy of fusion of the pasty compound is the amunt of energy necessary to make the compound change from the solid state to the liquid state. It is expressed in J/g.

The enthalpy of fusion consumed at 23°C is the amount of energy absorbed by the sample to change from the solid state to the state which it exhibits at 23°C, consisting of a liquid fraction and a solid fraction.

The liquid fraction of the pasty compound measured at 32°C preferably represents from 30% to 100% by weight of the compound, preferably from 50% to 100%, more preferably from 60% to 100% by weight of the compound. When the liquid fraction of the pasty compound measured at 32°C is equal to 100%, the temperature of the end of the melting range of the pasty compound is less than or equal to 32°C.

The liquid fraction of the pasty compound measured at 32°C is equal to the ratio of the enthalpy of fusion consumed at 32°C to the enthalpy of fusion of the pasty compound. The enthalpy of fusion consumed at 32°C is calculated in the same way as the enthalpy of fusion consumed at 23°C.

As regards the measurement of the hardness, the sample preparation and measurement protocols are as follows: The pasty fatty substance is placed in a mould with a diameter of 75 mm which is filled to approximately 75% of its height. In order to overcome the thermal history and to control the crystallization, the mould is placed in a Vötsch VC 0018 programmable oven, where it is first of all placed at a temperature of 80°C for 60 minutes, then cooled from 80°C to 0°C at a cooling rate of 5°C/minute, then left at the stabilized temperature of 0°C for 60 minutes, then subjected to a temperature rise passing from 0°C to 20°C at a heating rate of 5°C/minute and then left at the stabilized temperature of 20°C for 180 minutes.

The compressive force measurement is taken using a TA/TX2i texture analyzer from Swantech. The spindle used is chosen according to the texture:
- cylindrical steel spindle 2 mm in diameter for very rigid starting materials;
- cylindrical steel spindle 12 mm in diameter for relatively non-rigid starting materials.

The measurement comprises 3 stages: a first stage after automatic detection of the surface of the sample, where the spindle moves at a measuring speed of 0.1 mm/s and penetrates into the pasty fatty substance to a penetration depth of 0.3 mm, the software notes the value of the maximum force achieved; a second "relaxation" stage where the spindle remains at this position for one second and where the force is noted after 1 second of relaxation; finally, a third "withdrawal" stage where the spindle returns to its initial position at a speed of 1 mm/s and the probe withdrawal energy (negative force) is noted.

The value of the hardness measured during the first stage corresponds to the maximum compressive force measured in newtons divided by the surface area of the cylinder of the texture analyzer in contact with the pasty fatty substance, expressed in mm². The hardness value obtained is expressed in megapascals or MPa.

The pasty fatty substance is preferably chosen from synthetic compounds and compounds of vegetable origin. A pasty compound can be obtained by synthesis from starting products of vegetable origin.

The pasty compound is advantageously chosen from:
- lanolin and its derivatives;
- petrolatum, in particular the product which has this as INCI name and which is sold under the name Ultima White
   PET USP by Penreco;
- polyol ethers chosen from polyalkylene glycol pentaerythrityl ethers, fatty alcohol ethers of sugars, and their mixtures, polyethylene glycol pentaerythrityl ether comprising five oxyethylene (5 OE) units (CTFA name: PEG-5 Pentaerythrityl Ether), polypropylene glycol pentaerythrityl ether comprising five oxypropylene (5 OP) units (CTFA name: PPG-5 Pentaerythrityl Ether), and their mixtures, and more especially the PEG-5 Pentaerythrityl Ether, PPG-5 PentaerythritylEther and soybean oil mixture sold under the Lanolide name by Vevy, which is a mixture in which the constituents are in a 46/46/8 ratio by weight: 46% PEG-5 Pentaerythrityl Ether, 46% PPG-5 Pentaerythrityl Ether and 8% soybean oil;
- polymeric or non-polymeric silicone compounds;
- polymeric or non-polymeric fluorinated compounds;
- vinyl polymers, in particular:
   - olefin homopolymers and copolymers,
   - hydrogenated diene homopolymers and copolymers,
   - linear or branched oligomers which are homopolymers or copolymers of alkyl (meth)acrylates preferably having a C8-C30 alkyl group,
   - oligomers which are homopolymers and copolymers of vinyl esters having C8-C30 alkyl groups,
   - oligomers which are homopolymers and copolymers of vinyl ethers having C8-C30 alkyl groups;
- fat-soluble polyethers resulting from the polyetherification between one or more C2-C100 and preferably C2-C50 diols;
- esters;
- and/or their mixtures.

The pasty compound is preferably a polymer, in particular a hydrocarbon polymer.

Preference is given in particular, among the fat-soluble polyethers, to copolymers of ethylene oxide and/or of propylene oxide with long-chain C6-C30 alkylene oxides, more preferably such that the ratio by weight of the ethylene oxide and/or of the propylene oxide to the alkylene oxides in the copolymer is from 5:95 to 70:30. In this family, mention will be made in particular of copolymers such that the long-chain alkylene oxides are positioned in blocks having an average molecular weight from 1000 to 10 000, for example a polyoxyethylene/polydodecyl glycol block copolymer, such as the ethers of dodecanediol (22 mol) and of polyethylene glycol (45 OE) sold under the brand name Elfacos ST9 by Akzo Nobel.

Preference is given in particular, among the esters, to:
- esters of an oligomeric glycerol, in particular diglycerol esters, especially condensates of adipic acid and of glycerol, for which a portion of the hydroxyl groups of the glycerols have reacted with a mixture of fatty acids, such as stearic acid, capric acid, stearic acid and isostearic acid, and 12-hydroxystearic acid, preferably such as bis-diglyceryl polyacyladipate-2, sold under the brand name Softisan 649 by Sasol,
- arachidyl propionate, sold under the brand name Waxenol 801 by Alzo,
- phytosterol esters,
- fatty acid triglycerides and their derivatives, such as, for example, triglycerides of fatty acids, in particular C10-C18
   fatty acids, which are partially or completely hydrogenated, such as those sold under the reference Softisan100 by Sasol,
- pentaerythritol esters, and their mixtures,
- esters of a diol dimer and of a diacid dimer, where appropriate esterified on their free alcohol or acid functional group(s) with acid or alcohol radicals, in particular dimer dilinoleate esters; such esters can be chosen in particular from the esters having the following INCI nomenclature: bis-behenyl/isostearyl/phytosteryl dimer dilinoleyl dimer dilinoleate (Plandool G), phytosteryl/isostearyl/cetyl/stearyl/behenyl dimer dilinoleate (Plandool H or Plandool S), and their mixtures,
- mango butter, such as the product sold under the reference Lipex 203 by AarhusKarlshamn,
- shea butter, in particular the product for which the INCI name is Butyrospermum Parkii Butter, such as the product sold under the reference Sheasoft^{®} by AarhusKarlshamn,
- and their mixtures,

The choice will preferably be made, among the pasty compounds, of bis-behenyl/isostearyl/phytosteryl dimer dilinoleyl dimer dilinoleate, bis-diglyceryl polyacyladipate-2, hydrogenated castor oil dimer dilinoleate, for example Risocast DA-L sold by Kokyu Alcohol Kogyo, hydrogenated castor oil isostearate, for example Salacos HCIS (V-L) sold by Nisshin Oil, mango butter, shea butter, vinylpyrrolidone/eicosene copolymers, or their mixture(s).

A composition according to the invention is preferably devoid of pasty fatty substance. However, a composition according to the invention can comprise one or more pasty fatty substances at a total content of greater than or equal to 0.01 % by weight, with respect to the total weight of the composition, for example between 0.1 % and 5% by weight, with respect to the total weight of the composition.

### Emulsifying system according to the invention

The compositions according to the invention comprise a main emulsifying system comprising at least one anionic surfactant **in the salt form chosen from C16-C18 monoalkyl phosphate,** at least one cationic counterion and at least one basic amino acid.

The term "main emulsifying system" is understood to mean a system which, in its absence, does not result in the formation of a stable composition.

The term "stable" is advantageously understood to mean a composition which, after having been placed in an oven at 45°C for two months, does not exhibit, after returning to ambient temperature, grains perceptible to the touch when a thin layer of the composition is sheared between the fingers.

### Anionic surfactant(s) in the salt form

Each of these said anionic surfactants can be used alone or as a mixture with one or more other said anionic surfactants.

The designation of monoalkyl is understood to mean that the phosphate element is combined with just one ***C16-C18*** alkyl chain, unless otherwise specified. The monoalkyl phosphate(s) (including phosphine oxide(s)) which can be used in the compositions according to the present patent application is/are chosen C16-C18 monoalkyl phosphates, and their mixtures.

Preferably, they are chosen from monocetyl phosphate, monostearyl phosphate and monocetearyl phosphate.

It is in particular monocetyl phosphate, for example sold under the names Amphisol K (Roche), Amphisol A (Roche), Arlatone MAP (Uniqema) and Crodafos MCA (Croda).

Preferably, the total content of anionic surfactant(s) and the total content of wax(es) are such that the ratio by weight of anionic surfactant(s) to the wax(es) is greater than or equal to 1/10, preferably between 1/8 and 1/2 inclusive.

In particular, this (these) anionic surfactant(s) in accordance with the invention can be present at a content of greater than or equal to 1% by weight, with respect to the total weight of the composition, preferably of greater than or equal to 3% by weight, with respect to the total weight of the composition. Generally, this (these) anionic surfactant(s) in accordance with the invention can be present at a content ranging from 1.5% to 15% by weight and better still from 2% to 10% by weight, with respect to the total weight of the composition.

### Cationic counterion(s)

The anionic surfactant(s) in accordance with the invention comprise(s) a cationic counterion.

This cationic counterion can be of inorganic origin, in particular chosen from alkali metal cations, or of organic origin; preferably, it is of inorganic origin.

The alkali metal cation(s) can be chosen from sodium and potassium.

The cation(s) of organic origin can be chosen from ammonium and its amine and aminoalcohol derivatives. Preferably, the cation(s) of organic origin is (are) chosen from ammonium and its amine and aminoalcohol derivatives.

According to a specific embodiment, the cation is a primary (poly)hydroxyalkylamine. The term "primary (poly) hydroxyalkylamine" is understood in particular to mean a primary dihydroxyalkylamine, it being understood that the term "primary" is understood to mean a primary amine functional group, i.e. -NH2, and the alkyl group is a linear or branched C1-C8 hydrocarbon chain, preferably a branched C4 hydrocarbon chain, such as 1,3-dihydroxy-2-methylpropyl. The primary (poly)hydroxyalkylamine is preferentially 1,3-dihydroxy-2-methyl-2-propylamine (also known as aminomethylpropanediol or AMPD).

According to a preferred embodiment, the cationic counterion comprises at least one alkali metal cation, this cation comprising at least potassium.

The total content of cationic counterion(s) and in particular of potassium is preferably greater than or equal to 0.05%, in particular inclusively between 0.08% and 3% by weight and better still between 0.5% and 2% by weight, with respect to the total weight of the composition.

### Basic amino acid(s)

The basic amino acid(s) in accordance with the invention is (are) preferably chosen from arginine, histidine, lysine and their mixture(s).

This (these) basic amino acid(s) can be in the L or D isomer form, preferably in the L isomer form.

According to a preferred embodiment, the basic amino acid is arginine, preferably L-arginine.

According to the invention, the total content of basic amino acid(s) and the total content of anionic surfactant(s), in particular of monocetyl phosphate, are such that the ratio by weight of the basic amino acid(s) to the anionic surfactant(s) is greater than or equal to 0.05, preferentially ranges from 0.075 to 10, preferentially ranges from 0.085 to 2 and more preferentially still ranges from 0.1 to 0.5.

The total content of basic amino acid(s) is greater than or equal to 0.1% by weight, with respect to the total weight of the composition, and preferentially ranges from 0.5% to 4% by weight, with respect to the total weight of the composition;

### Co-surfactant(s)

A composition according to the invention advantageously comprises one or more co-surfactants.

A composition according to the invention advantageously comprises at least one co-surfactant chosen from fatty alcohols comprising from 10 to 26 carbon atoms, better still from 12 to 24 carbon atoms and even better still from 14 to 22 carbon atoms.

The total content of co-surfactant(s) is preferably inclusively between 0.01% and 5% by weight, with respect to the total weight of the composition.

### Additional surfactant(s)

A composition according to the invention can comprise one or more additional surfactant(s) distinct from the emulsifying system described above.

According to a specific embodiment, a composition according to the invention does not comprise additional surfactant(s).

Preferably, a composition according to the invention does not comprise cationic or amphoteric additional surfactant(s).

According to a specific embodiment, a composition in accordance with the invention can comprise at least one additional surfactant chosen from non-ionic surfactants.

A preferred emulsifying system of the invention comprises:
- at least one C12-C22 monoalkyl phosphate, in particular a monocetyl phosphate, present at a content of greater than or equal to 5% by weight, with respect to the total weight of the composition,
- at least one alkali metal counterion present at a content of greater than or equal to 0.01% by weight, with respect to the total weight of the composition, in particular a potassium counterion, and
- at least one basic amino acid, in particular arginine, present at a content of greater than or equal to 0.1 % by weight, with respect to the total weight of the composition.

According to a preferred specific embodiment, the cosmetic composition according to the present invention does not comprise C16-C22 fatty acid in the acid form, in particular, does not comprise stearic acid.

According to a preferred embodiment, the cosmetic composition according to the present invention comprises less than 1% and preferably less than 0.5% by weight of triethanolamine and better still is devoid of triethanolamine.

According to a specific embodiment, the cosmetic composition according to the present invention comprises less than 1% and preferably less than 0.5% by weight of triethanolamine stearate and better still is devoid of triethanolamine stearate.

Preferably, a composition according to the invention comprises less than 2% by weight of additional emulsifying system, with respect to the total weight of the composition, indeed even less than 1% by weight, and more preferentially is devoid of additional surfactant. In particular, a composition according to the invention is preferably devoid of additional ionic surfactant, in particular of additional anionic surfactant and of additional non-ionic surfactant.

### Film-forming polymer

The compositions according to the present patent application preferably comprise at least one hydrophilic or lipophilic, preferably hydrophilic, film-forming polymer.

In the present patent application, the term "film-forming polymer" is understood to mean a polymer which is capable, by itself alone or in the presence of an additional film-forming agent, of forming a macroscopically continuous deposited layer and preferably a cohesive deposited layer, better still a deposited layer having cohesive and mechanical properties such that the said deposited layer can be isolated and handled in isolation, for example when the said deposited layer is prepared by pouring onto a non-stick surface, such as a Teflon-coated or silicone-coated surface.

Generally, the content as dry matter of "film-forming polymer" of the compositions according to the present patent application ranges from 0.1% to 40%, preferably from 0.5% to 30% and better still from 1% to 10% by weight, with respect to the total weight of the composition.

The hydrophilic film-forming polymer can be a water-soluble polymer or be provided in dispersion in an aqueous medium.

Mention may be made, among the film-forming polymers which can be used in the composition of the present invention, of synthetic polymers, of radical type or of polycondensate type, polymers of natural origin and their mixtures.

Mention may be made, as examples of water-soluble film-forming polymers, of:
- proteins, such as proteins of vegetable origin, for example wheat or soybean proteins, or proteins of animal origin,
   such as keratins, for example keratin hydrolysates and sulfonic keratins;
- cellulose polymers, such as hydroxyethylcellulose, hydroxypropylcellulose, methylcellulose, ethylhydroxyethylcellulose or carboxymethylcellulose, and also quaternized cellulose derivatives;
- acrylic polymers or copolymers, such as polyacrylates or polymethacrylates;
- vinyl polymers, such as polyvinylpyrrolidones, copolymers of methyl vinyl ether and of malic anhydride, the copolymer of vinyl acetate and of crotonic acid, copolymers of vinylpyrrolidone and of vinyl acetate, copolymers of vinylpyrrolidone and of caprolactam, or polyvinyl alcohol;
- anionic, cationic, amphoteric or non-ionic chitin or chitosan polymers;
- gums arabic, guar gum, xanthan derivatives, karaya gum or acacia gum;
- alginates and carrageenans;
- glycoaminoglycans, hyaluronic acid and its derivatives;
- shellac resin, sandarac gum, dammars, elemis or copals;
- deoxyribonucleic acid;
- mucopolysaccharides, such as chondroitin sulfates;
   and their mixtures.

The film-forming polymer can also be present in the composition in the form of particles dispersed in an aqueous phase, generally known under the name of latex or pseudolatex. The techniques for preparing these dispersions are well known to a person skilled in the art.

Use may also made, as aqueous dispersion of film-forming polymer, of the acrylic dispersions sold under the names Neocryl XK-90^{®}, Neocryl A-1070^{®}, Neocryl A-1090^{®}, Neocryl BT-62^{®}, Neocryl A-1079^{®} and Neocryl A-523^{®} by Avecia-Neoresins, Dow Latex 432^{®} by Dow Chemical, Daitosol 5000 AD^{®} or Daitosol 5000 SJ^{®} by Daito Kasey Kogyo; Syntran 5760^{®} by Interpolymer or Allianz Opt^{®} by Rohm and Haas or alternatively the aqueous polyurethane dispersions sold under the names Neorez R-981^{®} and Neorez R-974^{®} by Avecia-Neoresins, Avalure UR-405^{®}, Avalure UR-410^{®}, Avalure UR-425^{®}, Avalure UR-450^{®}, Sancure 875^{®}, Avalure UR-445^{®} and Sancure 2060^{®} by Noveon, Impranil 85^{®} by Bayer, Aquamere H-1511^{®} by Hydromer; the sulfopolyesters sold under the brand name Eastman AQ^{®} by Eastman Chemical Products, vinyl dispersions, such as Mexomere PAM^{®}, aqueous dispersions of polyvinyl acetate,such as Vinybran^{®} from Nisshin Chemical or those sold by Union Carbide, aqueous dispersions of vinylpyrrolidone/dimethylaminopropylmethacrylamide/lauryldimethylpropylmethacr ylamidoammonium chloride, such as Styleze W from ISP, aqueous dispersions of hybrid polyurethane/polyacrylic polymers, such as those sold under the references Hybridur^{®} by Air Products or Duromer^{®} from National Starch, core/shell dispersions: for example, those sold by Atofina under the reference Kynar (core: fluorinated - shell: acrylic) or else those described in the document US 5 188 899 (core; silica - shell: silicone) and their mixtures.

A composition according to the invention can also comprise, in an alternative form or additionally, a lipophilic polymer which can be in solution or in dispersion in a non-aqueous solvent phase.

### Colorants

The compositions in accordance with the invention comprise at least one colorant.

This (or these) colorant(s) is (are) preferably chosen from pulverulent colorants, fat-soluble dyes, water-soluble dyes and their mixtures.

Preferably, the compositions according to the invention comprise at least one pulverulent colorant. The pulverulent colorants can be chosen from pigments and pearlescent agents, preferably from pigments.

The pigments can be white or coloured, inorganic and/or organic and coated or uncoated. Mention may be made among the inorganic pigments, of metal oxides, in particular titanium dioxide, optionally surface-treated, zirconium, zinc or cerium oxide, and also iron, titanium or chromium oxide, manganese violet, ultramarine blue, chromium hydrate and ferric blue. Mention may be made, among the organic pigments, of carbon black, pigments of D & C type and lakes based on cochineal carmine of barium, strontium, calcium or aluminium.

The pearlescent agents can be chosen from white pearlescent pigments, such as mica covered with titanium dioxide or with bismuth oxychloride, coloured pearlescent pigments, such as titanium oxide-coated mica with iron oxides,
titanium oxide-coated mica with in particular ferric blue or chromium oxide, or titanium oxide-coated mica with an organic pigment of the abovementioned type, and also pearlescent pigments based on bismuth oxychloride.

The fat-soluble dyes are, for example, Sudan red, D&C Red 17, D&C Green 6, β-carotene, soybean oil, Sudan brown, D&C Yellow 11, D&C Violet 2, D&C Orange 5, quinoline yellow and annatto.

Preferably, the pigments present in the compositions according to the invention are chosen from metal oxides.

These colorants can be present in a content ranging from 0.01% to 30% by weight, with respect to the totalweight of the composition, in particular from 3% to 15% by weight, with respect to the total weight of the composition.

Preferably, the colorant(s) is (are) chosen from one or more metal oxides present at a content of greater than or equal to 2% by weight, with respect to the total weight of the composition, advantageously inclusively between 3% and 15% by weight, with respect to the total weight of the composition.

### Fillers

The compositions in accordance with the invention can also comprise at least one filler.

The fillers can be chosen from those well known to a person skilled in the art and commonly used in cosmetic compositions. The fillers can be inorganic or organic and lamellar or spherical. Mention may be made of talc, mica, silica, kaolin, powders formed of polyamide, such as Nylon^{®} sold under the name Orgasol^{®} by Atochem, poly-β-alanine and polyethylene, powders formed of tetrafluoroethylene polymers, such as Teflon^{®}, lauroyllysine, starch, boron nitride, expanded hollow polymeric microspheres, such as those of polyvinylidene chloride/acrylonitrile, for example the products sold under the name Expancel^{®} by Nobel Industrie, acrylic powders, such as those sold under the name Polytrap^{®} by Dow Corning, polymethyl methacrylate particles and silicone resin microbeads (for example Tospearls^{®} from Toshiba), precipitated calcium carbonate, magnesium carbonate, basic magnesium carbonate, hydroxyapatite, hollow silica microspheres (Silica Beads^{®} from Maprecos), glass or ceramic microcapsules, or metal soaps derived from organic carboxylic acids having from 8 to 22 carbon atoms and in particular from 12 to 18 carbon atoms, for example zinc stearate, magnesium stearate, lithium stearate, zinc laurate or magnesium myristate.

Use may also be made of a compound capable of swelling on heating and in particular of heat-expandable particles, such as non-expanded microspheres of vinylidene chloride/acrylonitrile/methyl methacrylate copolymer or of acrylonitrile homopolymer or copolymer, such as, for example, those respectively sold under the references Expancel^{®} 820 DU 40 and Expancel^{®} 007WU by Akzo Nobel.

The fillers can represent from0. 1 % to 25% by weight and in particular from 0.2% to 20% by weight, with respect to the total weight of the composition.

### Fibres

The compositions in accordance with the invention can also comprise at least one fibre which makes it possible to improve the lengthening effect.

In the case of the preparation of a top coat comprising fibres intended to cover a base coat composition in accordance with the invention, the inventors were able to observe that the non-volatile oil according to the invention facilitated the placing of the fibres at the end of the natural eyelashes.

The term "fibre" should be understood as meaning an object of length L and of diameter D such that L is very much greater than D, D being the diameter of the circle in which the cross section of the fibre is inscribed. In particular, the ratio L/D (or aspect ratio) is chosen within the range from 3.5 to 2500, in particular from 5 to 500 and more particularly from 5 to 150.

The fibres which can be used in the composition of the invention can be inorganic or organic fibres of synthetic or natural origin. They can be short or long, individual or organized, for example braided, and hollow or solid. They can have any shape and can in particular have a circular or polygonal (square, hexagonal or octagonal) cross section, depending on the specific application envisaged. In particular, their ends are blunted and/or polished to prevent injury.

In particular, the fibres have a length ranging from 1 mm to 10 mm, in particular from 0.1 mm to 5 mm and more particularly from 0.3 mm to 3.5 mm. Their cross section can be included in a circle with a diameter ranging from 2 nm to 500 mm, in particular ranging from 100 nm to 100 mm and more particularly ranging from 1 mm to 50 mm. The weight or count of fibres is often given in denier or decitex and represents the weight in grams per 9 km of yarn. The fibres according to the invention can in particular have a count chosen within the range from 0.15 to 30 denier and in particular
from 0.18 to 18 denier.

The fibres which can be used in the composition of the invention can be chosen from rigid or non-rigid fibres and they can be inorganic or organic fibres of synthetic or natural origin.

Moreover, the fibres may or may not be surface-treated, may or may not be coated and may or may not be coloured.

Mention may be made, as fibres which can be used in the composition according to the invention, of non-rigid fibres, such as polyamide (Nylon^{®}) fibres, or rigid fibres, such as polyimideamide fibres, for example those sold under
the names Kermel^{®} and Kermel Tech^{®} by Rhodia, or poly(p-phenylene terephthalamide) (or aramid) fibres, sold in
particular under the name Kevlar^{®} by DuPont de Nemours.

The fibres can be present in the composition according to the invention in a content ranging from 0.01% to 10% by weight, in particular from 0.1% to 5% by weight and more particularly from 0.3% to 3% by weight, with respect to the total weight of the composition.

### Cosmetic active agents

The compositions in accordance with the invention can also comprise at least one cosmetic active agent.

Mention may in particular be made, as cosmetic active agents which can be used in the compositions in accordance with the invention, of antioxidants, preservatives, fragrances, neutralizing agents, emollients, thickeners, coalescence agents, plasticizers, moisturizing agents, vitamins and screening agents, in particular sunscreens, and their mixtures.

Of course, a person skilled in the art will take care to choose the optional additional additives and/or their amount so that the advantageous properties of the composition according to the invention are not, or not substantially, detrimentally affected by the envisaged addition.

Preferably, the composition according to the invention is a leave-in composition. Advantageously, the composition is a make-up composition and in particular a mascara.

### ASSEMBLY

An assembly for coating keratinous fibres suitable for the invention can comprise an applicator configured in order to apply the said cosmetic composition for coating keratinous fibres and, where appropriate, a packaging device suitable for receiving the said composition.

### Applicator

The applicator can comprise means which make it possible to smooth and/or separate keratinous fibres, such as the eyelashes or eyebrows, in particular in the form of teeth, bristles or other protruding parts.

The applicator is designed in order to apply the composition to the eyelashes or eyebrows and can comprise, for example, a brush or a comb.

The applicator can also be used for finishing the make-up, over a region of the eyelashes or eyebrows which is made up or laden with the composition.

The brush can comprise a twisted core and bristles held between the turns of the core or can be made in yet another way.

The comb is, for example, produced from a single part by moulding plastic.

In some implementational examples, the application element is mounted at the end of a rod which can be flexible, which can contribute to improving the comfort during application.

### Packaging device

The packaging device can comprise a container intended to house the composition for coating keratinous fibres. This composition can then be withdrawn from the container by immersing the applicator in it.

This applicator can be integral with an element for closing the container. This closing element can form a member for grasping the applicator. This grasping member can form a cap to be removably mounted on the said container by any suitable means, such as screwing, snap-fastening, push-fitting or other. Such a container can thus reversibly house the said applicator.

This container can optionally be equipped with a wiper suitable for removing a surplus of product withdrawn by the applicator.

A method for applying thecomposition according to the invention to the eyelashes or eyebrows can also comprise the following stages:
- forming a deposited layer of the cosmetic composition on the eyelashes or eyebrows,
- leaving the deposited layer on the eyelashes or eyebrows, it being possible for the deposited layer to dry.

It should be noted that, according to another embodiment, the applicator can form a product container. In such a case, a container can, for example, be provided in the grasping member and an internal channel can internally connect this grasping member to the protruding application elements.

Finally, it should be noted that the packaging and application assembly can be provided in the form of a kit, it being possible for the applicator and the packaging device to be housed separately in one and the same packaging article.

The preceding and following examples are given by way of illustration of the present invention and cannot limit the scope thereof.

### EXAMPLES

### 1/ Preparation of a mascara composition according to the invention compared with a mascara composition outside the invention

| Phase | Ingredients with percentage contents | Composition according to the invention | Comparative composition outside the invention 1 | Comparative composition outside the invention 2 |
|---|---|---|---|---|
| F | Carnauba wax | 9 | 9 | 9 |
| F | Beeswax | 8 | 8 | 8 |
| F | Paraffin wax | 13 | 13 | 13 |
| F | Pigment | 8 | 8 | 8 |
| A | Water | q.s. for 100 | q.s. for 100 | q.s. for 100 |
| A | Hydroxyethylcellulose | 1 | 1 | 1 |
| A | Acacia gum | 4 | 4 | 4 |
| A | Potassium cetyl phosphate (Amphisol K from DSM Nutritional Products) | 8 | 8 | 0 |
| A | Stearic acid | 0 | 0 | 8 |
| A | L-Arginine (from Ajinomoto) | 1 | 0 | 1 |
| A | Preserving system | q.s. | q.s. | q.s. |

These compositions were prepared as follows:

### i. Preparation of the fatty phase (F)

The various waxes are melted in a 500 ml jacketed heating pan with circulation of hot oil to control the temperature. The mixture is heated to approximately 96-98°C.

When the waxes are molten and homogenized, the pigments are added and the mixture is then homogenized using a Moritz stirrer (stirring of rotor-stator type).

### ii. Preparation of the aqueous phase (A)

A 600 ml tall-form beaker is used. The amount of water necessary, preheated in an electric kettle, is stirred slowly in this beaker using a Rayneri mixer.

The (co)polymers and then the surfactants are successively introduced, still with slow stirring. Between each introduction, care is taken to ensure good dissolution of the compound and homogenization of the medium.

The preservatives are then added.

The aqueous phase is then placed on a water bath (set at 90-92°C) until a temperature of 88-90°C is reached.

### iii. Emulsification

When the two phases are at the desired temperature, the aqueous phase is added very slowly to the fatty phase while gradually increasing the stirring. The stirring is maintained for 10 minutes.

### iv. Temperature reduction

After the emulsification, the heating pan is placed on a Rayneri mixer equipped with a butterfly paddle, which allows blending and homogenization during the temperature reduction, at low shear. The stirring speed is low so as not to incorporate air bubbles.

By means of an oil bath, the temperature is gradually reduced to ambient temperature (20°C): in stationary phases of 10°C.

### v. End of formulation

The mascara thus obtained is transferred into a closed jar in order to prevent it from drying on contact with the air; it is then necessary to wait 24 hours in order to confirm that the formulation is homogenous and that the pigments are correctly dispersed.

### 2/ Protocols and Results

The compositions prepared are subsequently observed under a microscope and are then used for making up, by application of these compositions using a brush.

For the comparative composition outside the invention 1, a mascara having a grey colour, with a very thick texture, which cannot be used for making up or packaged, is observed.

For the comparative composition outside the invention 2, a mascara having a grey colour, with a very thick texture, which cannot be used for making up or packaged, is observed.

For the composition according to the invention, a creamy, smooth and black texture is observed. The emulsion of the waxes is fine and even. The pigments are well dispersed. The composition obtained is nice and shiny. It is pleasant to apply and comfortable. The make-up result is smooth and even. The eyelash fringe is well-developed. In addition, these compositions are stable at 4 and 45°C for two months.

### 2/ Preparation of a mascara composition according to the invention compared with a mascara composition outside the invention (determination of criticality of a ratio of 0.05 of the basic amino acid(s) to the anionic surfactant(s))

| Phase | Ingredients with percentage contents | Composition according to the invention | Comparative composition outside the invention 3 |
|---|---|---|---|
| F | Carnauba wax | 9 | 9 |
| F | Beeswax | 8 | 8 |
| F | Paraffin wax | 13 | 13 |
| F | Pigment | 8 | 8 |
| A | Water | q.s. for 100 | q.s. for 100 |
| A | Hydroxyethylcellulose | 1 | 1 |
| A | Acacia gum | 4 | 4 |
| A | Potassium cetyl phosphate (Amphisol K from DSM Nutritional Products) | 8 | 8 |
| A | Stearic acid | 0 | 0 |
| A | L-Arginine (from Ajinomoto) | 0.4 | 0.2 |
| A | Preserving system | q.s. | q.s. |

The same preparation protocol as described above was carried out in order to obtain the above compositions.

Comparative composition 3, which exhibits a ratio by weight of the basic amino acid(s) to the anionic surfactant(s) which is less than 0.05, gives rise to a very coarse and non-homogeneous mascara, contrary to a test in which the ratio is 0.05 or more.

It is understood that, in the context of the present invention, the percentages by weight given for a compound or a family of compounds are always expressed as weight of dry matter of the compound in question.

Throughout the patent application, the wording "comprising a" means "comprising at least one", unless otherwise specified.

## Claims

1. Cosmetic composition for coating keratinous fibres of the emulsion type, comprising:
- an aqueous phase,
- at least one wax, wherein the wax(es) is (are) present at a total content of greater than or equal to 20% by weight, with respect to the total weight of the composition and
- an emulsifying system comprising:
i) at least one anionic surfactant in the salt form chosen from C16-C18 monoalkyl phosphate,
ii) at least one cationic counterion, and
iii) at least one basic amino acid,
in which the total content of basic amino acid(s) and the total content of anionic surfactant(s) in the salt form are such that the ratio by weight of the basic amino acid(s) to the anionic surfactant(s) in the salt form is greater than or equal to 0.05.

2. Composition according to Claim 1 comprising at least one non-polar wax and at least one polar wax.

3. Composition according to Claim 1 or 2, in which the said composition comprises at least 20% by weight of nonpolar wax(es) and preferably at least 40% by weight of non-polar wax(es), with respect to the total weight of wax(es).

4. Composition according to any one of the preceding claims, in which the said emulsifying system comprises at least one monocetyl phosphate.

5. Composition according to any one of the preceding claims, in which the total content of anionic surfactant(s) is greater than or equal to 1% by weight, with respect to the total weight of the composition, preferably between 1.5% and 15% by weight, with respect to the total weight of the composition.

6. Composition according to any one of the preceding claims, in which the total content of anionic surfactant(s) and the total content of wax(es) are such that the ratio by weight of anionic surfactant(s) to the wax(es) is greater than or equal to 1/10, preferably between 1/8 and 1/2 inclusive.

7. Composition according to any one of the preceding claims, in which the cationic counterion(s) is (are) chosen from cation(s) of inorganic or organic origin, preferably of inorganic origin, preferably chosen from alkali metal cations and more preferably comprising potassium.

8. Composition according to any one of the preceding claims, in which the basic amino acid(s) is (are) preferably chosen from arginine, histidine, lysine and their mixture(s), and preferably comprises arginine.

9. Composition according to any one of the preceding claims, in which the total content of basic amino acid(s) is greater than or equal to 0.1 % by weight, with respect to the total weight of the composition, and preferentially ranges from 0.5% to 4% by weight, with respect to the total weight of the composition.

10. Composition according to any one of the preceding claims, comprising at least one hydrophilic film-forming polymer.

11. Composition according to any one of the preceding claims, comprising at least one colorant chosen from one or more pulverulent colorant(s), preferably metal oxides and in particular iron oxides.

12. Method for coating keratinous fibres, in particular for making up the eyelashes, comprising a stage of applying a cosmetic composition for coating keratinous fibres according to any one of the preceding claims.

## Patentansprüche

1. Kosmetische Zusammensetzung zum Beschichten von keratinischen Fasern vom Emulsionstyp, umfassend:
- eine wässrige Phase,
- mindestens ein Wachs, wobei das Wachs bzw. die Wachse in einem Gesamtgehalt größer gleich 20 Gew.-%, bezogen auf das Gesamtgewicht der Zusammensetzung, vorliegt bzw. vorliegen, und
- ein Emulgiersystem, umfassend:
i) mindestens ein anionisches Tensid in der Salzform, das aus C₁₆-C₁₈-Monoalkylphosphat ausgewählt ist,
ii) mindestens ein kationisches Gegenion und
iii) mindestens eine basische Aminosäure,
wobei der Gesamtgehalt von basischer Aminosäure bzw. basischen Aminosäuren und der Gesamtgehalt von anionischem Tensid bzw. anionischen Tensiden in der Salzform so beschaffen sind, dass das Gewichtsverhältnis von basischer Aminosäure bzw. basischen Aminosäuren zu anionischem Tensid bzw. anionischen Tensiden in der Salzform größer gleich 0,05 ist.

2. Zusammensetzung nach Anspruch 1, die mindestens ein unpolares Wachs und mindestens ein polares Wachs umfasst.

3. Zusammensetzung nach Anspruch 1 oder 2, wobei die Zusammensetzung mindestens 20 Gew.-% unpolares Wachs bzw. unpolare Wachse und vorzugsweise mindestens 40 Gew.-% unpolares Wachs bzw. unpolare Wachse, bezogen auf das Gesamtgewicht von Wachs bzw. Wachsen, umfasst.

4. Zusammensetzung nach einem der vorhergehenden Ansprüche, wobei das Emulgiersystem mindestens ein Monocetylphosphat umfasst.

5. Zusammensetzung nach einem der vorhergehenden Ansprüche, wobei der Gesamtgehalt von anionischem Tensid bzw. anionischen Tensiden größer gleich 1 Gew.-%, bezogen auf das Gesamtgewicht der Zusammensetzung, beträgt und vorzugsweise zwischen 1,5 und 15 Gew.-%, bezogen auf das Gesamtgewicht der Zusammensetzung, liegt.

6. Zusammensetzung nach einem der vorhergehenden Ansprüche, wobei der Gesamtgehalt von anionischem Tensid bzw. anionischen Tensiden und der Gesamtgehalt von Wachs bzw. Wachsen so beschaffen sind, dass das Gewichtsverhältnis von anionischem Tensid bzw. anionischen Tensiden zu Wachs bzw. Wachsen größer gleich 1/10 ist und vorzugsweise zwischen 1/8 und 1/2 inklusive liegt.

7. Zusammensetzung nach einem der vorhergehenden Ansprüche, wobei das kationische Gegenion bzw. die kationischen Gegenionen aus einem Kation bzw. Kationen anorganischer oder organischer Herkunft, vorzugsweise anorganischer Herkunft, das bzw. die vorzugsweise aus Alkalimetallkationen ausgewählt ist bzw. sind und weiter bevorzugt Kalium umfasst bzw. umfassen, ausgewählt ist bzw. sind.

8. Zusammensetzung nach einem der vorhergehenden Ansprüche, wobei die basische Aminosäure bzw. die basischen Aminosäuren vorzugsweise aus Arginin, Histidin, Lysin und Mischung(en) davon ausgewählt ist bzw. sind und vorzugsweise Arginin umfasst.

9. Zusammensetzung nach einem der vorhergehenden Ansprüche, wobei der Gesamtgehalt von basischer Aminosäure bzw. basischen Aminosäuren größer gleich 0,1 Gew.-%, bezogen auf das Gesamtgewicht der Zusammensetzung, ist und vorzugsweise im Bereich von 0,5 bis 4 Gew.-%, bezogen auf das Gesamtgewicht der Zusammensetzung, liegt.

10. Zusammensetzung nach einem der vorhergehenden Ansprüche, die mindestens ein hydrophiles filmbildendes Polymer umfasst.

11. Zusammensetzung nach einem der vorhergehenden Ansprüche, die mindestens ein Farbmittel umfasst, das aus einem oder mehreren pulverförmigen Farbmitteln, vorzugsweise Metalloxiden und insbesondere Eisenoxiden, ausgewählt ist.

12. Verfahren zum Beschichten von keratinischen Fasern, insbesondere zum Schminken der Wimpern, bei dem man eine kosmetische Zusammensetzung zum Beschichten von keratinischen Fasern nach einem der vorhergehenden Ansprüche aufbringt.

## Revendications

1. Composition cosmétique pour le revêtement de fibres kératiniques du type émulsion, comportant :
- une phase aqueuse,
- au moins une cire, **la ou les cires étant présentes à raison d'une teneur totale supérieure ou égale à 20** % **en poids, par rapport au poids total de la composition** et
- un système émulsionnant comprenant :
i) au moins un agent tensioactif anionique sous la forme de sel choisi parmi du phosphate de monoalkyle en C16-C18,
ii) au moins un contre-ion cationique, et
iii) au moins un acide aminé basique,
dans laquelle la teneur totale en acide(s) aminé(s) basique(s) et la teneur totale en agent(s) tensioactif (s) anionique (s) sous la forme de sel sont telles que le rapport pondéral du ou des acide(s) aminé(s) basique(s) sur le ou les agent(s) tensioactif (s) anionique(s) sous la forme de sel est supérieur ou égal à 0,05.

2. Composition selon la revendication 1, comportant au moins une cire non polaire et au moins une cire polaire.

3. Composition selon la revendication 1 ou 2, dans laquelle ladite composition comprend au moins 20 % en poids de cire (s) non polaire (s) et de préférence au moins 40 % en poids de cire(s) non polaire (s), par rapport au poids total de cire(s).

4. Composition selon l'une quelconque des revendications précédentes, dans laquelle ledit système émulsionnant comprend au moins un phosphate de mono-cétyle.

5. Composition selon l'une quelconque des revendications précédentes, dans laquelle la teneur totale en agent(s) tensioactif(s) anionique(s) est supérieure ou égale à 1 % en poids, par rapport au poids total de la composition, de préférence entre 1,5 % et 15 % en poids, par rapport au poids total de la composition.

6. Composition selon l'une quelconque des revendications précédentes, dans laquelle la teneur totale en agent(s) tensioactif(s) anionique(s) et la teneur totale en cire(s) sont telles que le rapport pondéral en agent(s) tensioactif(s) anionique(s) sur la ou les cire(s) est supérieur ou égal à 1/10, de préférence inclusivement compris entre 1/8 et 1/2.

7. Composition selon l'une quelconque des revendications précédentes, dans laquelle le ou les contre-ion(s) cationique(s) est/sont choisi(s) parmi un ou des cation(s) d'origine inorganique ou organique, de préférence d'origine inorganique, de préférence choisi(s) parmi les cations de métaux alcalins, et plus préférentiellement comprenant le potassium.

8. Composition selon l'une quelconque des revendications précédentes, dans laquelle le ou les acide(s) aminé(s) basique(s) est/sont de préférence choisi(s) parmi l'arginine, l'histidine, la lysine, et leur(s) mélange(s), et de préférence comprend/comprennent l'arginine.

9. Composition selon l'une quelconque des revendications précédentes, dans laquelle la teneur totale en acide(s) aminé(s) basique(s) est supérieure ou égale à 0,1 % en poids par rapport au poids total de la composition, et préférentiellement dans la plage de 0,5 % à 4 % en poids, par rapport au poids total de la composition.

10. Composition selon l'une quelconque des revendications précédentes, comprenant au moins un polymère filmogène hydrophile.

11. Composition selon l'une quelconque des revendications précédentes, comprenant au moins une matière colorante choisie parmi une ou plusieurs matière(s) colorante(s) pulvérulente(s), de préférence des oxydes métalliques, et en particulier des oxydes de fer.

12. Procédé pour le revêtement de fibres kératiniques, en particulier pour le maquillage des cils, comprenant une étape d'application d'une composition cosmétique pour le revêtement de fibres kératiniques selon l'une quelconque des revendications précédentes.
